(19) **Europäisches Patentamt**

**European Patent Office**

**Office européen des brevets**

(11) Publication number: **0 057 590**
A2

# (12) EUROPEAN PATENT APPLICATION

(21) Application number: **82300474.2**

(22) Date of filing: **29.01.82**

(51) Int. Cl.³: **B 29 C 25/00, A 61 F 1/00,
D 06 C 11/00**

(30) Priority: **30.01.81 JP 13612/81**

(43) Date of publication of application: **11.08.82**
**Bulletin 82/32**

(84) Designated Contracting States: **AT BE DE FR IT NL SE**

(71) Applicant: **JUNKOSHA CO. LTD., 42-1, 1-chome
Gotokuji, Setagaya-ku Tokyo 154 (JP)**

(72) Inventor: **Sato, Toshikazu Junkosha Musashino-ryo,
322 Umahikizawa hidaka-machi, Iruma-gun Saitama-ken
(JP)**

(74) Representative: **Taylor, Derek George et al, MATHISEN,
MACARA & CO. European Patent Attorneys Lyon House
Lyon Road, Harrow Middlesex, HA1 2ET (GB)**

(54) **Napped fluororesin material having continuous pores, and a method of manufacturing the same.**

(57) A sheet or tube of fluororesin material having conti-
nuous pores, particularly polytetrafluoroethylene, has a nap
raised on at least one surface thereof, and is suitable for
prosthetic use as a patch, or an artificial blood vessel. A
method of manufacturing such napped fluororesin material
comprises impregnating fluororesin material having continuous pores with water, freezing it, raising a nap thereon,
and defrosting and dewatering it.

EP 0 057 590 A2

NAPPED FLUORORESIN MATERIALS HAVING
CONTINUOUS PORES, AND A METHOD OF
MANUFACTURING THE SAME

This invention relates to fluororesin materials
having continuous pores, and which are used for the prosthesis
of a malfunctioning heart or other organs, or blood
vessels, or the manufacture of filters, clothes, or the
like.

Sheets or tubes of fluororesins having continuous
pores, particularly polytetrafluoroethylene (PTFE), are
widely used for making patches, artificial blood vessels,
or other medical substitutes, various types of filters,
diaphragms, or water-repellent, air-permeable clothes.
These products are all very effective. Such PTFE material having continuous pores can be made by a method as
disclosed in Japanese Patent Publication No. 18991/1976.
According to this method, an admixture of fine PTFE powder
and a liquid lubricant is preformed into a paste, the paste
is extruded through a ram extruder, and the extruded product
is stretched in a selected. direction at a stretch rate of
at least 10% per second at a temperature lower than the
melting point of PTFE crystals (about 327°C). If required,
the material may be heated at a temperature higher than
327°C, while it is held against shrinkage, to provide a
baked product. Products having different shapes and physical properties can be obtained if the shape of the product obtained by extrusion, the stretch ration and the heating temperature are varied. These stretched porous PTFE (hereinafter called EPTFE) products comprise numerous fine knots united together by fibrils, and defining numerous fine continuous pores having a diameter of, say, 0.1 to 5 microns.The tubular products may be used individually or stacked together to provide artificial blood vessels, while the membraneous products may be laminated on other material to make various types of filters or clothes, or the like.

None of the known products has, however, been completely satisfactory. Some materials for prosthetic use comprise EPTFE alone, while others comprise a combination of EPTFE and other woven or knitted material, or plastic coating. They are available in tubular form for artificial blood vessels, and in sheet form for patches. The inner surface of the prosthetic material composed only of EPTFE, which is adapted to contact blood, is gradually covered after implantation with a false membrane spreading from the stitched region to form a structure which is comparable to the inner surface of a natural blood vessel in thrombosis resistance. The fibrils in a baked product are somewhat more likely to cause thrombosis than in an unbaked product. Both the baked and unbaked products have such smooth surfaces that the false membrane formed thereon is likely to peel away when some external impact has been applied thereto. If any such structure formed newly on the prosthetic material or any other matter adhering thereto peels away, the fragments thereof are likely to block finer blood vessels, resulting in serious thrombosis.

The outer surface of such prosthetic material, which is adapted to contact the body tissues, is also so smooth that no satisfactory adherence of the body tissues, or growth into the fine structure of the tissues takes place after implantation. Various kinds of prosthetic materials composed of EPTFE, and woven or knitted material have been proposed to improve the compatibility of EPTFE with the body tissues, and prevent the material from being torn by the stitching thread. These composite prosthetic materials are so reactive with the body tissues that they may cause thrombosis, or prevent the formation of a uniform false membrane. What is worse, serious thrombosis may result from the separation of fibers from the knitted material in the stitched region.

The smooth surface of EPTFE is difficult to dye. This is a great disadvantage of EPTFE for use as clothing material. It is not suitable for use as a facing for a laminate, but is mainly used to form an intermediate layer therein. A velvety surface would certainly give EPTFE a wider range of application in the field of clothing.

According to this invention, there is, thus, provided fluororesin material having continuous pores, and which is at least partly napped. The material of this invention may be manufactured by a method which essentially comprises impregnating fluororesin material having continuous pores with water, freezing it, raising a nap on the frozen material, defrosting the napped material, and dewatering it.

The material of this invention provides, for example, a very useful medical membrane, or patch which can form a uniform false membrane thereon quickly without undergoing any antibody reaction with any adjacent organ, and which does not present any problem without separating any foreign matter even if the incision is left open for a prolonged time. If it is used in the manufacture of clothing, its surface provides a velvety feel. The material of this invention is also suitable for making filters, and for many other purposes. The method of this invention is of great industrial value, since it facilitates economical napping on the continuously porous fluororesin material which is usually poor in workability.

## BRIEF DESCRIPTION OF THE DRAWINGS

FIGURE 1 is a fragmentary top plan view of an artificial blood vessel known in the art;

FIGURE 2 is a side elevational view of a sheet of prosthetic material known in the art;

FIGURE 3 is a side elevational view of a medical fluororesin membrane embodying this invention; and

FIGURE 4 is a fragmentary top plan view, partly in section, of a medical fluororesin tube embodying this invention, and stitched to a blood vessel.

## DETAILED DESCRIPTION OF THE INVENTION

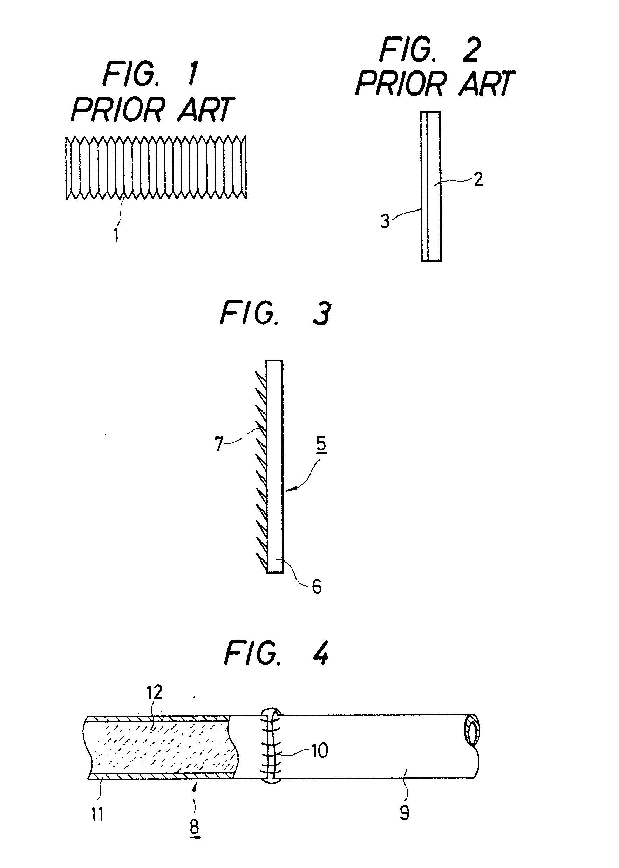

The prior art will be described more specifically with reference to FIGURES 1 and 2 of the drawings before this invention is described in detail. Referring to FIGURE 1, there is fragmentarily shown an artificial blood vessel 1 known in the art. The artificial blood vessel 1 comprises a tube of knitted fibers, and is stitched to a natural blood vessel. Although it is possible that a false membrane may grow about a core defined by the knitted fibers to form a relatively satisfactory new blood vessel, leakage of blood is often likely to occur during the stitching operation to cause an infant having only a small quantity of blood to die. Even if it does not die, some fibers are often likely to get separated and enter the finer blood vessel, and block it, resulting in serious thrombosis. It has, therefore, been proposed recently to employ a membrane of continuously porous fluororesin material having high thrombosis resistance, or any such membrane having a surface reinforced with knitted fibers.

Such a membrane or sheet of prosthetic material known in the art is shown in FIGURE 2. It comprises a membrane of fluororesin material having continuous pores such as polytetrafluoroethylene, and a layer of knitted fibers 3 bonded closely to one surface of the membrane 2. The knitted fibers 3 reinforce the membrane 2, and also assists the formation of a false membrane about the area in which prosthesis is achieved. This prosthetic material is, however, also unsatisfactory, since some of the fibers are often likely to get separated and enter the heart or blood vessel, resulting in thrombosis, or other trouble. In order to overcome these disadvantages, it has been proposed to eliminate the knitted fibers 3, and employ a membrane composed solely of fluororesin material having a high degree of porosity and a very large pore diameter (i.e., a large fibril spacing). This attempt has, however, been unsuccessful, since a homogeneous membrane is difficult to manufacture, and the membrane does not permit fast growth of any uniform false membrane thereon.

According to this invention, a nap is raised on at least a portion of fluororesin material having continuous pores. The fluororesin material having continuous pores may, for example, comprise EPTFE obtained by stretching as hereinbefore described, a mixture thereof with a small quantity of other fluororesin, such as a copolymer of ethylene and tetrafluoroethylene, or tetrafluoroethylene and perfluoroalkylvinylether, porous material obtained by extracting an extractable powder from a mixture of fluororesin and the powder, or material obtained from fluororesin fibers by a papermaking process. The continuously porous fluororesin material obtained by stretching is most suitable. Such fluororesin material is too soft to be napped directly. It can, however, be napped economically, if it is impregnated with water, and frozen, and the frozen material is napped, defrosted and dewatered.

An unbaked sheet, tube or rod of polytetrafluoroethylene is prepared by a customary method, and stretched by a method as disclosed in Japanese Patent Publication No. 18991/1976, whereby it is formed with continuous pores.    While it is held against shrinkage, it is baked, whereby its strength is increased, and any creep phenomenon is prevented.    The fluororesin material having continuous pores is, then, impregnated with an alcohol having a low surface tension at ordinary room temperature.

The alcohol is, then, replaced with water.    Instead of any such alcohol, an aqueous solution of a surface active agent may be used as a liquid assistant having a low surface tension for the impregnation of the material with water.    Then, the material is placed in a freezer, and kept at a temperature which is sufficiently low to freeze the water, or usually in the range of -5°C to -30°C.    A nap can be raised on a surface of the frozen material if, for example, a rotary brush having relatively soft wires, such as of brass, is rubbed thereagainst.    The frozen and napped material can be defrosted if it is either left to stand at ordinary room temperature, or placed in a drying oven having a temperature lower than the melting point of the fluororesin material and not causing any shrinkage thereof, or in the range of, say, 80°C to 150°C.    Then, the material is dewatered and dried.

Referring now to FIGURE 3, there is shown a prosthetic membrane 5 formed from the continuously porous fluororesin material prepared as hereinabove described.    The membrane 5 comprises a sheet of continuous porous fluororesin material 6 having an outer surface on which a nap 7 is raised as hereinabove set forth.    The nap 7 may usually be formed by fibrils from the original film, and partly torn material, and have a length in the range of, say, 0.5 to 1,000 microns, depending on the purpose for which the membrane 5 is used.   As the nap 7 is raised uniformly on the outer surface of the fluororesin material 6, the prosthetic membrane 5 may be used successfully as, for example, a patch which does not undergo any antibody reaction with the human tissue, and forms a more uniform new tissue more quickly than the EPTFE material known in the art.   The prosthetic membrane prepared from the napped material according to this invention remains highly stable until after its implantation.

FIGURE 4 shows an artificial arterial tube 8 embodying this invention, and joined to a natural blood vessel 9 with a stitching thread 10.   The artificial arterial tube 8 comprises a stretched and baked polytetrafluoroethylene tube 11 having an inner surface on which a nap 12 is uniformly formed.   The nap can be provided on the outer surface of the tube 11, or both the inner and outer surfaces thereof.   The nap 12 facilitates fast and uniform growth of a false membrane, and enables the artificial blood vessel to remain safe even if the incision is left open for a prolonged time.

Although the invention has been described with reference to the preferred embodiments thereof, it is to be understood that modifications or variations may be easily made by anybody of ordinary skill in the art without departing from the scope of this invention which is defined by the appended claims.

0057590

<u>CLAIMS</u>

1. Fluororesin material having continuous pores therein, characterised by a nap extending over at least a part of the surface of said material.

2. Fluororesin material according to claim 1 characterised in that it is in the form of a sheet having the nap on at least one side thereof.

3. Fluororesin material according to claim 1 characterised in that it is in the form of a tube having said nap on at least one of the inner and outer surfaces of the tube.

4. Fluororesin material according to any preceding claim characterised in that it comprises polytetrafluoroethylene.

5. Fluororesin material according to claim 1 characterised in that it is in the form of a patch for prosthetic use.

6. Fluororesin material according to claim 3 characterised in that it is in the form of an artificial blood vessel.

7. A method of manufacturing fluororesin material according to any of the preceding claims which is characterised by the steps of:

impregnating the fluororesin material with water;

freezing said water;

raising a nap on said material;

defrosting and removing the water from said material.


8. A method according to claim 7 characterised by impregnating said material with a liquid having a low surface tension, and selected from the group consisting of alcohols and surface active agents before said material is impregnated with water, said liquid being then replaced by said water.


9. A method according to claim 7 or claim 8 characterised by raising the nap by brushing said material with a soft wire brush.

0057590

FIG. 1
PRIOR ART

1

FIG. 2
PRIOR ART

2

3

FIG. 3

7

5

6

FIG. 4

12

10

11

8

9